(19) Europäisches Patentamt
European Patent Office
Office européen des brevets

(11) Veröffentlichungsnummer: **0 192 845**
**B1**

(12) # EUROPÄISCHE PATENTSCHRIFT

(45) Veröffentlichungstag der Patentschrift: **15.02.89**

(51) Int. Cl.⁴: **C 07 C 15/44**, C 07 C 1/30, C 07 C 5/25

(21) Anmeldenummer: **85115998.8**

(22) Anmeldetag: **14.12.85**

(54) **Verfahren zur Herstellung von reinem Methallylbenzol bzw. Isobutenylbenzol und ihren p-substituierten Alkyl-Derivaten.**

(30) Priorität: **15.02.85 DE 3505156**

(43) Veröffentlichungstag der Anmeldung: **03.09.86 Patentblatt 86/36**

(45) Bekanntmachung des Hinweises auf die Patenterteilung: **15.02.89 Patentblatt 89/7**

(84) Benannte Vertragsstaaten: **AT BE CH DE FR GB IT LI NL SE**

(56) Entgegenhaltungen:
**DD-A-99 358**
**US-A-2 454 779**

(73) Patentinhaber: **HÜLS AKTIENGESELLSCHAFT, - RSP Patente / PB 15 - Postfach 13 20, D-4370 Marl 1 (DE)**

(72) Erfinder: **Kaufhold, Manfred, Dr., Jasminweg 20, D-4370 Marl (DE)**

**EP 0 192 845 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft ein Verfahren zur Herstellung von reinem Methallylbenzol bzw. Isobutenylbenzol und ihren p-substituierten Alkyl-Derivaten der Formel 1 bzw. 2 durch thermische Spaltung von Neophylchlorid bzw. eines entsprechenden p-substituierten Neophylchlorids der Formel 3 und gegebenenfalls anschließender Isomerisierung der Spaltolefine der Formel 2 unter den Bedingungen einer fraktionierten Destillation.

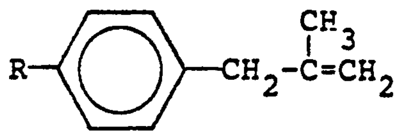

1

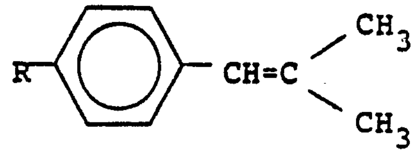

2

R = H, $-CH_3$, $CH_3CH_2-$,

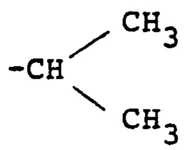
,
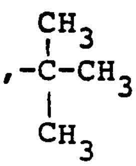

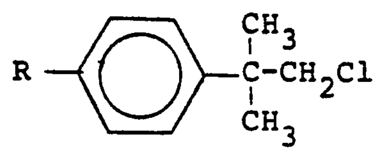

3

Synthesen von Methallylbenzol, Isobutenylbenzol und ihren p-substituierten Alkyl-Derivaten, die von dem leicht zugänglichen Neophylchlorid bzw. seinen Derivaten ausgehen, sind literaturbekannt.

So beschrieben bereits Whitmore, Weisgerber und Shabica (s. Am. Soc. 65 (1943), 1469 1470) die thermische Spaltung des Neophylchlorids zum Methallylbenzol und Isobutenylbenzol; sie erhielten ein Pyrolyseprodukt mit folgender Zusammensetzung:

| | |
|---|---|
| Methallylbenzol | 23,5 % |
| Isobutenylbenzol | 36,5 % |
| Neophylchlorid | 26,0 % |
| 1-Phenyl-2.2-dimethyl-ethylchlorid | 8,9 % |

Eine verbesserte Ausbeute von 83 % an Olefingemisch wird mit einem Verfahren der DD-PS 99 358 erreicht, bei dem nicht umgesetztes Neophylchlorid immer wieder in die Spaltungsapparatur zurückgeführt wird. Wie das Beispiel zeigt, enthält das Pyrolyseprodukt neben nicht umgesetztem Neophylchlorid relativ viel Rückstand (mehr als 10 % der Menge an Olefingemisch).

Die Herstellung und Spaltung von p-substituierten Alkyl-Derivaten des Neophylchlorids beschreibt die US-PS-2 454 779. Im Gegensatz zu den beiden zuerst genannten Arbeiten ist es das ausdrückliche Ziel dieses Verfahrens, die thermische Spaltung so zu lenken, daß Methallylbenzol bzw. seine Derivate und nicht wie bei den o. g. Literaturbeispielen Isobutenylbenzol bzw. dessen Derivate als Hauptprodukt entstehen. Denn die α-Olefine haben als Ausgangsprodukte zur Herstellung von Riechstoffen erheblich größere Bedeutung als ihre Isomeren, die β-Olefine.

Die gewünschte Beeinflußung des Isomerenverhältnisses wird in der o. g. US-PS dadurch erreicht, daß man die Pyrolyse in Gegenwart von stöchiometrischen Mengen eines Alkalisalzes einer Carbonsäure durchführt. Die erhaltenen Isomerenverhältnisse liegen nach Angaben der Beispiele ungefähr bei α : β-Olefin wie 3 : 1 bis 4 : 1. Auch im günstigsten Fall entsteht also das unerwünschte Nebenprodukt in großer Menge und macht deshalb dieses Verfahren unwirtschaftlich. Das Arbeiten mit den Alkalisalzen von Carbonsäuren hat zudem den Nachteil, daß kostbarer Reaktorraum verbraucht wird und führt zum Anfall von Natriumchlorid, das als Abfall beseitigt werden muß.

Verfahren zur Isomerisierung des Isobutenylbenzols zum Methallylbenzol sind bisher nicht bekannt.

Im allgemeinen werden für derartige Isomerisierungen, bei denen es um eine Verschiebung der Doppelbindungen geht, verschiedenartige Katalysatoren eingesetzt.

So werden z. B. bei einem Verfahren zur Stellungsisomerisierung von endständigen Doppelbindungen in Olefinen (EP-PS-0 042 537) stark saure Kationenaustauscher als Katalysatoren verwendet. Dagegen arbeitet das Verfahren der DE-OS-3 319 171, bei dem Buten-2 zu Buten-1 isomerisiert wird, d. h. bei einer ähnlichen Gleichgewichtsreaktion wie in der EP-PS-0 042 537, mit speziellen Metalloxiden der Gruppen II A und/oder VIII und/oder III B auf γ-Tonerde.

Alle bekannten Verfahren liefern somit Olefingemische, benötigen deshalb einen hohen Materialeinsatz und

führen zu Problemen mit der Abfallbeseitigung. Wünschenswert wäre ein Verfahren, bei dem das technisch durch Umsetzung von Methallylchlorid mit dem entsprechenden Alkylaromaten leicht zugängliche p-Alkylneophylchlorid ohne Anfall von Abwasser in ein Olefingemisch überführt würde, aus diesem das wertvolle α-Olefin z. B. durch Destillation isoliert und das nicht so wertvolle β-Olefine in das α-Olefin umgewandet werden könnte.

Es besteht ein großes Interesse an einem Verfahren, nach dem man bei geringem Materialverbrauch aus Neophylchlorid bzw. aus dem jeweiligen p-Alkylneophylchlorid reines Methallylbenzol bzw. p-Alkylmethallylbenzol herstellen kann, weil diese α-Olefine wichtige Rohstoffe zur Produktion von Riechstoffen wie z. B. p-tert.-Butyl-α-methyldihydrozimtaldehyd (auch Lilial genannt) sind.

Die sich hieraus ergebende Aufgabe wird erfindungsgemäß entsprechend den Angaben der Patentansprüche gelöst.

Überraschenderweise erhält man bei der thermischen Spaltung von Neophylchlorid und p-Alkylneophylchloriden wesentlich bessere Ausbeuten als z. B. in der o. g. DD-PS 99 358 angegeben, wenn man die Spaltung in Gegenwart eines Inhibitors für die radikalische Polymerisation durchführt.

Außerdem erhält man Methallylbenzol bzw. p-Alkylmethallylbenzol auf überraschend einfache Weise aus dem Spaltolefingemisch, indem man durch Zusatz von Alkalien und einem polaren Lösemittel eine Isomerisierung bewirkt und mittels fraktionierter Destillation das gewünschte niedriger siedende α-Olefin ausschleust.

Überraschenderweise hat sich gezeigt, daß durch die für die Isomerisierung getroffenen Maßnahmen eine weitere Reinigung der Olefine bewirkt wird, indem der Restchlorgehalt von z. B. 200 bis 300 ppm auf unter 10 ppm gesenkt wird. Die Verringerung des Chlorgehaltes ist wegen Korrosionsgefahr eine entscheidende Voraussetzung für die Weiterverarbeitung der erfindungsgemäß gewonnenen α-Olefine in Apparaturen, die aus den handelsüblichen Werkstoffen wie z. B. V4A bestehen.

Die Verbesserung der Ausbeute insbesondere des Umsatzes bei der Spaltung von Neophylchlorid und p-Alkyl-neophylchlorid durch Zusatz eines Inhibitors für die radikalische Polymerisation war überraschend, denn es war nur gegebenenfalls zu erwarten, daß der Anfall an Hochsiedern zurückging und nicht, daß die rein thermische Spaltung dadurch begünstigt würde. Nach dem erfindungsgemäßen Verfahreh werden Umsätze an Neophylchlorid von über 95 % und Ausbeuten an Olefingemisch, bezogen auf Umsatz, von mindestens 98 % erreicht, während im Beispiel 1 der o. g. DD-PS 99 358 nur eine Ausbeute an Olefingemisch von 66,5 %, bezogen auf eingesetztes Neophylchlorid, genannt wird. Bei der Spaltung des p-tert.-Butylneophylchlorids liegen die Umsätze beim erfindungsgemäßen Verfahren noch höher und sind mit Werten von 99,9 % fast quantitativ.

Als Inhibitoren eignen sich Verbindungen, die üblicherweise zur Verhinderung von radikalischen Polymerisationen dienen, wie z. B. tert.-butyl-Brenzcatechin, Hydrochinon, 2,6-Di-tert.-Butylphenol usw. (bzgl. weiterer Inhibitoren s. Ullmanns Encyklopädie der technischen Chemie, Band 14, 3. Auflage, Verlage Urban & Schwarzenberg, München-Berlin 1963, S. 116).

Die Konzentration des Inhibitors beträgt 0,01 bis 5 %, vorzugsweise 0,1 bis 1 %. Die thermische Spaltung erfolgt bei 150 bis 350°C, vorzugsweise bei 180 bis 300°C. Die Spaltung führt man im allgemeinen bei Normaldruck durch, man kann aber auch im Über- bzw. Unterdruckbereich arbeiten. Die Umsetzung kann kontinuierlich und diskontinuierlich durchgeführt werden.

Ein wesentlicher Vorteil des erfindungsgemäßen Verfahrens ist die Isomerisierung des Olefingemisches bei gleichzeitiger destillativer Abtrennung des immer nachgebildeten α-Olefins. Dieser Syntheseschritt ist neu und löst die anfangs erläuterten Probleme des hohen Meterialverlustes, da anfallendes β-Olefin immer wieder in die Isomerisierung zurückgeführt werden kann.

Die bisher zur Isomerisierung von α- und β-Olefinen vorgeschlagenen Katalysatoren erwiesen sich bei der Isomerisierung des Isobutenylbenzols zum Methallylbenzol, also auch der Isomerisierung eines β-Olefins zu einem α-Olefin, als ungeeignet, da sie nicht effektiv genug sind. Überraschenderweise wurden sehr gute Ergebnisse mit stark basischen Katalysatorsystemen erzielt. Als stark basische Katalysatorsysteme eignen sich Gemische aus einer oder mehreren basischen Verbindungen wie z. B. Alkalihydroxiden, LiOH, NaOH, KOH, RbOH und CsOH, vorzugsweise Natrium und/oder Kaliumhydroxid, und einem polaren Lösemittel wie z. B. Alkohole, Diole, Polyole, Glykolether und/oder eine polare aprotische Verbindung. Bevorzugt setzt man als polares Lösemittel Alkohole wie Methanol, Ethanol, Propanol, Isopropanol, insbesondere höhersiedende Alkohole wie Isobutanol, tert.-Butanol, n-Butanol, n- oder iso-Amylalkohole ein. Als polare aprotische Verbindungen eignen sich vorzugsweise Dimethylsulfoxid, Dimethylformamid, N-Methyl-pyrolidon, Alkylglykolether mit $C_1$- bis $C_4$-Alkylgruppen, beispielsweise Butyldiglykol und Butyltriglykol, die man insbesondere gemeinsam mit Natriumhydroxid als basische Verbindung einsetzt, Polyglykole und/oder Polyethylenglykoldiether der allgemeinen Formel $R\text{-}O(\text{-}CH_2\text{-}CH_2O)_n\text{-}R$ mit $n = 1$ bis 20 und $R = C_{1\text{-}4}$-Alkyl.

Die basische Verbindung setzt man in Mengen von 0,1 bis 50 %, bezogen auf Olefingemisch, vorzugsweise von 0,5 bis 5, ein.

Das polare Lösemittel setzt man im allgemeinen in Mengen von 1 bis 50 %, bezogen auf Olefingemisch, ein, vorzugsweise von 1 bis 10 %.

Diese stark basischen Katalysatorsysteme wurden bisher nur bei einer chemisch andersartigen Isomerisierung, der Isomerisierung von isolierten zu konjugierten Doppelbindungen in niedermolekularen Homo- und/oder Copolymeren von 1,3-Dienen, eingesetzt (DE-OSS-3 205 990, 3 227 684 und 3 227 685).

Die Isomerisierung erfolgt im allgemeinen bei Temperaturen von 80 bis 300°C, vorzugsweise bei 180 bis

300°C. Gegebenenfalls setzt man auch bei der Isomerisierung die bei der Spaltung verwendeten Inhibitoren ein.

Das erfindungsgemäße Verfahren führt man beispielsweise folgendermaßen durch:

Ausgangsprodukt des erfindungsgemäßen Verfahrens ist Neophylchlorid bzw. ein p-Alkyl-Neophylchlorid, das man im allgemeinen aus Benzol bzw. einem Alkylbenzol und Methallylchlorid in Gegenwart von Schwefelsäure als Katalysator herstellt (bzgl. Neophylchlorid siehe z. B. Organic Syntheses, Collective Volume 4 (Annual V. 30 bis 39), John Wiley & Sons Inc., 1963, S. 702); bzgl. p-Alkyl-Neophylchlorid-Synthesen siehe o. g. US-PS-2 454 779) und anschließend durch Destillation oder Umkristallisieren reinigt. Die thermische Spaltung wird bei Normaldruck oder etwas erhöhtem Druck, beispielsweise bei 1 bis 20 bar, vorzugsweise 1 bis 5 bar, entweder in Form einer Destillation durchgeführt, wobei die leichtsiedenden Spaltprodukte mit dem gebildeten gasförmigen Chlorwasserstoff "über Kopf gehen" und das nicht umgesetzte Neophylchlorid bzw. p-Alkyl-Neophylchlorid durch den eingestellten Rückfluß in den heißen Sumpf zurückgeführt wird, oder die Chlorverbindung wird in Substanz oder in einem Lösemittel erhitzt, wobei die Spaltprodukte aber nicht der entstehende Chlorwasserstoff durch einen Kühler zurückgehalten werden.

Die Sumpftemperaturen liegen zwischen 150 bis 350°C, vorzugsweise bei 180 bis 300°C. Vor der Spaltung wird zu der eingesetzten Chlorverbindung ein Inhibitor bzw. Stabilisator zugesetzt, der üblicherweise zur Verhinderung von radikalischen Polymerisationen dient wie z. B. tert.-Butyl-Brenzcatechin. Die Konzentration des Inhibitors bzw. Stabilisators beträgt 0,01 bis 5 %, vorzugsweise 0,1 bis 1 %.

Falls erforderlich, werden die Spaltolefine durch Destillation gereinigt und vom nicht umgesetzten Ausgansprodukt abgetrennt.

Die Umisomerisierung der Spaltolefine erfolgt in einer Destillationsapparatur, wobei ein Lösemittel z. B. Isobutanol und eine starke Base z. B. Kaliumhydroxid in den Sumpf der Apparatur gegeben werden. Die Konzentrationen dieser Zusätze betragen 1 bis 10 %, die angewandten Temperaturen liegen bei 80 bis 300°C, vorzugsweise 180 bis 300°C. Gegebenenfalls setzt man einen bei der Spaltung verwendeten Inhibitor zu. Wie bei jeder anderen Destillation richtet sich der anzuwendende Druck bei dem jeweiligen Produkt nach den gewünschten Temperaturen. So isomerisiert man beispielsweise das Gemisch von Methallylbenzol und Isobutenylbenzol bei Normaldruck, wobei sich Sumpftemperaturen von 185 bis 295°C und Kopftemperaturen von 92 bis 182°C einstellen. Dagegen liegen die Temperaturen bei der Isomerisierung von p-tert.-Butylisobutenylbenzol bei Normaldruck im Sumpf bei 232 bis 300°C und am Kopf bei 88 bis 241°C und bei einem Vakuum von 250 mbar werden Sumpftemperaturen von 196 bis 224°C und Kopftemperaturen von 64 bis 195°C gemessen. Den folgenden Beispielen ist weiteres Zahlenmaterial zu entnehmen; sie sollen das erfindungsgemäße Verfahren näher erläutern.

Die erfindungsgemäß hergestellten α-Olefine, Methallylbenzol oder p-Alkylmethallylbenzol eignen sich zur Herstellung von Riechstoffen wie beispielsweise p-tert.-Butyl-α-methyl-dihydrozimtaldehyd und sind wertvolle Zwischenprodukte für zahlreiche weitere technische Synthesen.

**Beispiel 1 a** - Thermische Spaltung

Man benutzt eine Glasapparatur, die aus einem Dreihalskolben mit Rührer, Thermometer und einer Destillationskolonne mit Destillationsaufsatz besteht.

Man setzt ein: 300 g Neophylchlorid (99,0 %-ig)
1 g tert.-Butyl-Brenzcatechin

Durch dieses Gemisch wird ein Stickstoffstrom mit einer Geschwindigkeit von 2 l/h geleitet. Dann wird die Temperatur auf 190°C erhöht und mit der Spaltung, die man anhand der Gasentwicklung erkennt, begonnen. Wie in der folgenden Tabelle angegeben, wird zunächst kein Produkt abdestilliert, sondern unter Rückfluß bis auf 210°C erhitzt.

Der weitere Temperaturverlauf ergibt sich aus der Tabelle.

| Reaktionszeit in h | Temperaturen (°C) Sumpf | Mantel | Kopf | Verhältnis Rückl. z.Abn. | $N_2$-Strom l/h |
|---|---|---|---|---|---|
| Beginn | 190 | 160 | - | keine Abn. | 2 |
| 0,5 | 212 | 160 | 172 | keine Abn. | 2 |
| 1 | 210 | 160 | 175 | keine Abn. | 2 |
| 1,5 | 208 | 160 | 178 | 5 : 1 | 2 |
| 2 | 212 | 160 | 181 | 5 : 1 | 2 |
| 2,5 | 213 | 160 | 182 | 5 : 1 | 2 |
| 3 | 214 | 160 | 182 | 5 : 1 | 2 |
| 3,5 | 215 | 160 | 178 | 5 : 1 | 2 |
| 4 | 216 | 160 | 173 | 5 : 1 | 2 |
| 4,5 | 212 | 160 | 182 | 5 : 1 | 2 |
| 5 | 212 | 160 | 181 | 5 : 1 | 2 |
| 5,5 | 228 | 160 | 180 | 20 : 1 | 2 |
| 6 | 248 | 160 | 178 | 20 : 1 | 2 |
| 6,5 | 296 | 160 | 180 | 20 : 1 | 2 |
| 7 | 300 | 160 | 180 | 20 : 1 | 2 |
| 7,5 | 296 | 175 | 178 | 20 : 1 | 2 |
| 8 | 296 | 175 | 172 | volle Abn. | 6 |
| 8,25 | 296 | 175 | 160 | Volle Abn. | 6 |

An Spaltprodukten fallen 232 g Destiiiat an, 7 g befinden sich in der Kolonne und 6 g verbleiben als Hochsieder im Sumpf.

Das neutral gewaschene Destillat hat einen Chlorgehalt von 1,9 % und zeigt nach GC-Analyse folgende Zusammensetzung:

| | |
|---|---|
| 1 Phenyl-2-methyl-propen-2 (1): | 33,4 % |
| 1-Phenyl-2-methyl-propen-1 (2): | 60,4 % |
| 1-Phenyl-2-methyl-2-chlorpropan: | 3,6 % |
| Neophylchlorid: | 1,9 % |
| nicht identifizierte Verbindungen: | 0,7 % |
| | 100,0 % |

Aus diesen Zahlen errechnet sich ein Umsatz an Chlorverbindungen zu Olefinen von 95,6 %. Auf den Umsatz bezogen beträgt die Ausbeute an Olefinen 98,2 %.

Die Reinigung der Olefine erfolgt durch Destillation in folgender Weise:

Einsatz: 217 g

| Fr. Nr. | Siedebereich (°C) | Druck (mbar) | Gew. (g) | Gew. (%) | Verhältnis Rückl. z. Abn. |
|---|---|---|---|---|---|
| 1 | 60 - 71 | 13 | 193 | 90,6 | 5 : 1 |
| Rückstand | | | 7 | 3,3 | |
| Kolonneninhalt | | | 11 | 5,2 | |
| Kühlfalle | | | 2 | 0,9 | |
| | | | 213 | 100,0 | |

(1) (Methallylbenzol)

(2) (Isobutenylbenzol)

Das Destillat hat einen Chlorgehalt von 800 ppm und nach GC-Analyse folgende Zusammensetzung:

| | |
|---|---|
| 1-Phenyl-2-methyl-propen-2 (1): | 35,3 % |
| 1-Phenyl-2-methyl-propen-1 (2): | 63,7 % |
| 1-Phenyl-2-methyl-2-chlorpropan: | 0,1 % |
| nicht identifizierte Verbindungen: | 0,9 % |
| | 100,0 % |

**Beispiel 1 b** - Isomerisierung

Man benutzt eine Glasapparatur, die aus einem Dreihalskolben mit Rührer, Thermometer und einer 0,5 m langen mit Multifil-Füllkörpern gefüllten Glaskolonne mit Destillationsaufsatz besteht.

Man setzt ein:

200 g destilliertes Olefingemisch mit Gehalten von 94,2 % an Isobutenylbenzol und 5,6 % an Methallylbenzol

10 g Isobutanol
4 g Kaliumhydroxid (ca. 90 %-ig)
0,2 g tert.-Butyl-Brenzcatechin

Das Gemisch wird unter Stickstoffabdeckung gerührt, auf 185°C erhitzt, nach 4stündigem Rühren bei dieser Temperatur wird Destillat abgenommen und in folgender Weise bei Normaldruck destilliert:

| Fr.-Nr. | Zeit (h) | Temperaturen (°C) Kopf | Sumpf | Gewicht (g) | (%) | Verhältnis Rückl. z. Abnahme |
|---|---|---|---|---|---|---|
| 1 | 2 | 92-175 | 185-186 | 10 | 4,8 | 10 : 1 |
| 2 | 6 | 176 | 186 | | | 20 : 1 |
| | | 178 | 186 | 178 | 84,8 | 10 : 1 |
| | | 178 | 224 | | | 20 : 1 |
| 3 | 1 | 182 | 292 | 14 | 6,7 | 5 : 1 |
| Rückstand | | | | 7 | 3,3 | |
| Kühlfalle | | | | 1 | 0,4 | |

(1) (Methallylbenzol)
(2) (Isobutenylbenzol)
Die Chlorgehalte der Fraktionen 2 bzw. 3 liegen bei 7 bzw. 10 ppm.

Die folgende Tabelle zeigt die Gehalte an Isobutanol und den Olefinen in den drei Fraktionen:
<u>GC-Analysenwerte (in %):</u>

| Verbindung | Franktions-Nr. 1 | 2 | 3 |
|---|---|---|---|
| Isobutanol | 89,0 | 0,1 | 0,1 |
| 1-Phenyl-2-methyl-propen-2 (1) | 9,5 | 75,4 | 19,7 |
| 1-Phenyl-2-methyl-propen-1 (2) | 0,5 | 24,0 | 80,0 |

Aus diesen Zahlen errechnet sich folgende Ausbeute:
Eingesetzt werden 188,4 g β-Olefin und 11,2 g α-Olefin. Erhalten werden 137,3 g α-Olefin und 54,0 g β-Olefin, d. h. neu gebildet werden 126,1 g α-Olefin oder 100 g β-Olefin (100 %-ig) ergeben 66,9 g α-Olefin.
Durch Redestillation werden die Isomeren in über 99 %-iger Reinheit erhalten.

**Beispiele 2 und 3** - Isomerisierung

Man benutzt die gleiche Apparatur wie im Beispiel 1 b beschrieben und verwendet die gleichen Ausgangsprodukte, setzt aber an Stelle von 10 g Isobutanol die gleiche Menge an tert.-Butanol bzw. bei einem weiteren Versuch 10 g Diglykol-n-butyl-tert.-butylether ein. Um gleich gute Ergebnisse wie beim Beispiel 1 b zu erzielen, muß die Isomerisierungszeit von 9 auf 20 bzw. 46 Stunden erhöht werden.
(1) (Methallylbenzol)
(2) (Isobutenylbenzol)

**Beispiel 4 a** - Thermische Spaltung

Man benutzt eine Glasapparatur, die aus einem Dreihalskolben (ohne Rührer) mit Thermometer, Gaseinleitungsrohr und Rückflußkühler besteht.
Man setzt ein: 259 g p-tert.-Butylneophylchlorid (98,8 %-ig, Schmelzpunkt 50 bis 52°C)
0,5 g tert.-Butyl-Brenzcatechin

Das Produktgemisch wird erwärmt und ein leichter Stickstoffstrom durch die Schmelze geleitet. Ab 218°C setzt die Spaltung ein. Nach einer halben Stunde liegt die Temperatur bei 238°C und nach einer Stunde bei 248°C. Diese Temperatur wird noch 6 h beibehalten, d. h. die gesamte Reaktionszeit beträgt 7 h.
Der Austrag hat einen Chlorgehalt von 240 ppm. Der Umsatz liegt demnach bei 99,9 %. Die Ausbeute an Olefinen beträgt 87 % d. Th., bezogen auf den Einsatz.
Nach gaschromatographischer Analyse enthält das Spaltprodukt
p-tert.-Butyl-Methallylbenzol zu 13,2 % und
p-tert.-Butyl-Isobutenylbenzol zu 78,7 %.

**Beispiel 4 b** - Isomerisierung

Man benutzt die im Beispiel 1 b beschriebene Apparatur und setzt folgende Produkte ein:
250 g Olefingemisch mit Gehalten von
78,8 % p-tert.-Butylisobutenylbenzol
19,5 % p-tert.-Butylmethallylbenzol
13 g Isobutanol
6,4 g Kaliumhydroxid (ca. 90 %-ig)
0,5 g tert.-Butyl-Brenzcatechin

Die Durchführung der Isomerisierung erfolgt bei Normaldruck wie im Beispiel 1 b beschrieben, wobei sich Sumpftemperaturen von 232 bis 300°C und Kopftemperaturen von 88 bis 240°C einstellen. Das gewünschte Produkt (218 g) siedet bei 238 bis 240°C, hat einen Chlorgehalt von nur 7 ppm und zeigt nach gaschromatographischer Analyse folgende Gehalte:

| | |
|---|---|
| p-tert.-Butylmethallylbenzol | 83,8 % |
| p-tert.-Butylisobutenylbenzol | 14,9 % |

Aus diesen Zahlen errechnet sich folgende Ausbeute:
Eingesetzt wird: 197 g β-Olefin und 48,8 g α-Olefin
Erhalten wird: 182,7 g α-Olefin und 32,5 g β-Olefin,
d. h. neu gebildet wird: 133,9 g α-Olefin oder
100,0 g β-Olefin (100 %-ig) ergeben
68,0 g α-Olefin

**Beispiel 5 a** - Thermische Spaltung

Man benutzt die im Beispiel 4 a beschriebene Apparatur und setzt zur Spaltung ein:
630 g p-Isopropylneophylchlorid (99,0 %-ig)
0,5 g tert.-Butyl-Brenzcatechin

Die Durchführung erfolgt wie im Beispiel 4 a beschrieben. Ab 264°C setzt die Chlorwasserstoffentwicklung ein und im Laufe der thermischen Spaltung geht infolge Bildung von Spaltprodukten die Temperatur im Kolben bis auf 236°C herunter. Das Reaktionsprodukt, 490 g, hat einen Chlorgehalt von 330 ppm; dieser entspricht einem Umsatz von 99,8 %. Durch Redestillation des rohen Spaltproduktes werden 35 g Hochsieder abgetrennt. Die Ausbeute an Olefingemisch beträgt 88,2 % d. Th., bezogen auf den Einsatz.

Nach gaschromatographischer Analyse enthält das Gemisch die Isomeren in folgenden Konzentrationen:

| | |
|---|---|
| p-Isopropylmethallylbenzol | 24,6 % |
| p-Isopropylisobutenylbenzol | 72,1 % |

**Beispiel 5 b** - Isomerisierung

Man benutzt die im Beispiel 1 b beschriebene Apparatur und setzt ein:
383 g destilliertes Olefingemisch mit Gehalten von
p-Isopropylmethallylbenzol 24,6 % und
p-Isopropylisobutenylbenzol 72,1 %
20 g Isobutanol
9,8 g Kaliumhydroxid (ca. 90 %-ig)
0,5 g tert.-Butyl-Brenzcatechin

Die Durchführung erfolgt wie im Beispiel 1 b beschrieben. Im Siedebereich von 227 bis 230°C bei Normaldruck werden 345 g einer Fraktion 2 und bei 234 bis 236°C werden 23 g einer Fraktion 3 mit folgenden Gehalten an α- bzw. β-Olefin erhalten:

| Verbindung | Fraktions-Nr. | |
|---|---|---|
| | 2 | 3 |
| p-Isopropylmethallylbenzol | 77,1 | 38,3 |
| p-Isopropylisobutenylbenzol | 18,0 | 61,1 |

Der Chlorgehalt der Fraktion 2 liegt bei 5 und der Chlorgehalt der Frakion 3 bei 8 ppm.

Aus den o. g. Zahlen errechnet sich folgende Ausbeute:

Eingesetzt werden 276,1 g β-Olefin und 94,2 g α-Olefin. Erhalten werden 274,8 g α-Olefin und 76,2 g β-Olefin, d. h. neu gebildet werden 180,6 g α-Olefin oder 100 g β-Olefin ergeben 65,4 g α-Olefin.

Durch Redestillation werden die Isomeren in über 99 %-iger Reinheit erhalten.

**Beispiel 6** - Thermische Spaltung

Man benutzt die im Beispiel 1 a beschriebene Apparatur und setzt folgende Produkte ein:

150 g p-Methylneophylchlorid (98,5 %-ig)
0,5 g Hydrochinon

Die Durchführung der thermischen Spaltung erfolgt bei Normaldruck wie im Beispiel 1 a beschrieben.

Der aus dem Chlorgehalt des Spaltproduktes errechnete Umsatz liegt bei 96,0 %. Auf den Umsatz bezogen beträgt die Ausbeute an Olefinen 97,0 %.

Nach Reinigung durch Destillation liegt der Chlorgehalt bei 530 ppm. Nach GC-Analyse enthält das Destillat die Isomeren in folgenden Konzentrationen:

p-Methylmethallylbenzol 31,2 %
p-Methylisobutenylbenzol 65,8 %

**Beispiel 7** - Thermische Spaltung

Man benutzt die im Beispiel 1 a beschriebene Apparatur und setzt folgende Produkte ein:

200 g p-Ehtylneophylchlorid (98,7 %-ig)
2 g 2,6-Di-tert.-Butylphenol

Die Durchführung der thermischen Spaltung erfolgt bei Normaldruck wie im Beispiel 1 a beschrieben.

Der aus dem Chlorgehalt des Spaltproduktes errechnete Umsatz liegt bei 97,1 %. Auf den Umsatz bezogen beträgt die Ausbeute an Olefinen 98,5 %.

Nach Reinigung durch Destillation liegt der Chlorgehalt bei 310 ppm.

Im Destillat liegen die Isomeren in folgenden Konzentrationen vor:

p-Ethylmethallylbenzol 30,7 %
p-Ethylisobutenylbenzol 66,4 %

**Beispiel 8** - Isomerisierung

Man benutzt die im Beispiel 1 b beschriebene Apparatur und setzt das hier genannte Olefingemisch mit folgenden Produkten ein:

250 g Olefingemisch
6 g Natriumhydroxid
30 g n-Butyldiglykol
30 g Isobutanol
0,5 g tert.-Butyl-Brenzcatechin

Die Durchführung erfolgt wie im Beispiel 1 b beschrieben. Das Ergebnis ist sehr ähnlich: Die Isomerisierungszeit beträgt ca. 10 Stunden. Das Destillat hat einen Chlorgehalt von 6 ppm. Aus 100 g β-Olefin entstehen 66,1 g α-Olefin.

**Patentansprüche**

1. Verfahren zur Herstellung von reinem Methallylbenzol bzw. Isobutenylbenzol und ihren p-substituierten Alkyl-Derivaten der Formel 1 bzw. 2 durch thermische Spaltung von Neophylchlorid bzw. eines entsprechenden

p-substituierten Neophylchlorids der Formel 3,
dadurch gekennzeichnet,
daß man die thermische Spaltung bei Temperaturen von 150 bis 350°C in Gegenwart eines Inhibitors für die radikalische Polymerisation durchführt und die erhaltenen Spaltolefine der Formel 2 durch frakionierte Destillation über ein Gemisch aus einer basischen Verbindung und einem polaren Lösemittel, gegebenenfalls in Gegenwart eines bei der Spaltung eingesetzten Inhibitors, bei Temperaturen von 80 bis 300°C zum α-Olefin der Formel 1 isomerisiert.

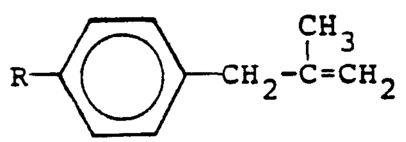

1

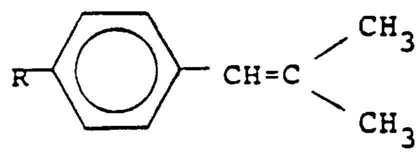

2

R = H, $-CH_3$, $CH_3CH_2-$,

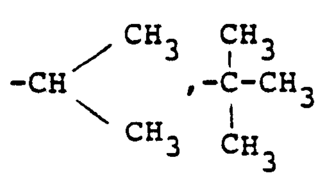

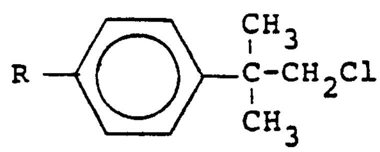

3

2. Verfahren nach Anspruch 1,
dadurch gekennzeichnet,
daß man als Inhibitor tert.-Butyl-Brenzcatechin, Hydrochinon oder 2,6-Di-tert.-Butylphenol einsetzt.

3. Verfahren nach den Ansprüchen 1 und 2,
dadurch gekennzeichnet,
daß man zur Isomerisierung als basische Verbindung Natrium- und/oder Kaliumhydroxid und als polare Lösemittel einen Alkohol und/oder eine polare aprotonische Verbindung einsetzt.

4. Verfahren nach den Ansprüchen 1 bis 3,
dadurch gekennzeichnet,
daß man als aprotische Verbindung Dimethylsulfoxid, Dimethylformamid, N-Methylpyrolidon, Alkylglykolether mit $C_1$- bis $C_4$-Alkylgruppen, Polyglykole, und/oder Polyethylenglykoldiether der allgemeinen Formel $R-O(-CH_2-CH_2O)_n-R$ mit n = 1 bis 20 und R = $C_{1-4}$-Alkyl einsetzt.

5. Verfahren nach den Ansprüchen 1 bis 3, dadurch gekennzeichnet, daß man die Isomerisierung bei Temperaturen von 180 bis 300°C durchführt.

**Claims**

1. A method for producing pure methallylbenzene or isobutenylbenzene and their p-substituted alkyl derivatives of formulas 1 and 2 respectively by thermal decomposition of neophyl chloride or a corresponding p-substituted neophyl chloride of formula 3, characterized in that thermal decomposition is performed at temperatures from 150 to 350°C in the presence of an inhibitor of polymerization by radicals, and the olefins of formula 2 obtained by cleavage are isomerized to α-olefins of formula 1 by fractional distillation over a mixture of a basic compound and a polar solvent at temperatures from 80 to 300°C, possibly in the presence of an inhibitor added during the decomposition

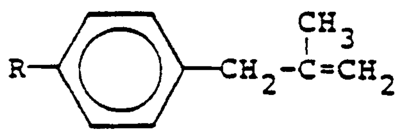

1

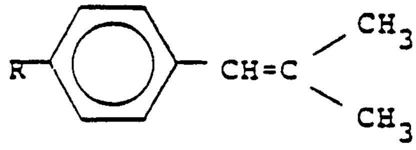

2

R = H, $-CH_3$, $CH_3CH_2-$,

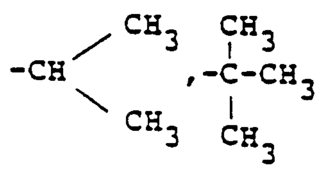

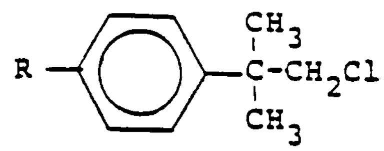

3

2. A method according to Claim 1, charàcterized in that tert.-butyl-pyrocatechin, hydroquinone or 2,6-di-tert.-butylphenol is used as inhibitor.

3. A method according to Claims 1 and 2, characterized in that sodium and/or potassium hydroxide is used as the basic compound and an alcohol and/or a polar aprotonic compound is used as the polar solvent.

4. A method according to Claims 1 to 3, characterized in that dimethylsulphoxide, dimethylformamide, N-methylpyrrolidone, alkylglycol ethers with $C_1$- to $C_4$-alkyl groups, polyglycols and/or polyethyleneglycol diethers with the general formula $R-O(CH_2-CH_2O)_n-R$, where n = 1 to 20 and R = $C_{1-4}$ alkyl, is used as the aprotonic compound.

5. A method according to Claims 1 to 3, characterized in that isomerization is performed at temperatures of from 180 to 300°C.

## Revendications

1. Procédé d'obtention de Méthallylbenzène ou d'Isobuténylbenzène purs et de leurs dérivés alcoylés p-substitués de formule 1 ou de formule 2 par coupure thermique du chlorure de Néophyle ou d'un chlorure de Néophyle p-substitué correspondant de formule 3, caractérisé en ce que l'on effectue la coupure thermique à des températures allant de 150 à 350°C en présence d'un inhibiteur de polymérisation radicalaire et que l'on isomérise l'oléfine de coupure de formule 2 obtenue, par distillation fractionnée au moyen d'un mélange à base d'un composé basique et d'un solvant polaire, le cas échant en présence d'un inhibiteur ajouté lors de la coupure à des températures allant de 80 à 300°C, en une α-oléfine de formule 1.

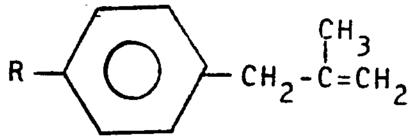

1

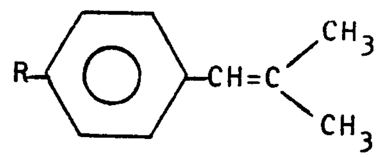

2

R = H, $-CH_3$, $CH_3CH_2-$,

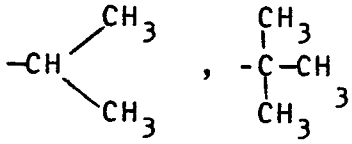

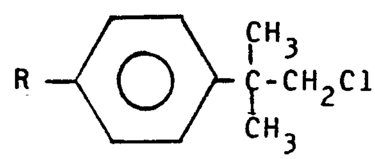

3

2. Procédé selon la revendication 1, caractérisé en ce que l'on met en oeuvre en tant qu'inhibiteur le terbutylpyrocatechol, l'hydroquinone ou le 2,6-diterbutylphénol.

3. Procédé selon les revendications 1 et 2, caractérisé en ce que l'on met en oeuvre pour l'isomérisation en

tant que composé basique l'hydroxyde de sodium et/ou de potassium et en tant que solvant polaire un alcool et/ou un composé polaire aprotique.

4. Procédé selon les revendications 1 à 3, caractérisé en ce que l'on met en oeuvre en tant que solvant polaire aprotique le diméthylsulfoxyde, le diméthylformamide, la N-méthylpyrolidone, un éther d'alcoylglycol ayant des groupes alcoyles en $C_1$-$C_4$ des polyglycols, et/ou un diéther de polyéthylèneglycol de formule générale

$R-O(-CH_2-CH_2O)_n-R$ avec n 1 à 20 et
R est un acoyle en $C_1$-$C_4$.

5. Procédé selon les revendications 1 à 3, caractérisé en ce que l'on effectue l'isomérisation à des températures allant de 180 à 300° C.